(19) Europäisches Patentamt
European Patent Office
Office européen des brevets

(11) Publication number: **0 060 787**
**A1**

(12) **EUROPEAN PATENT APPLICATION**

(21) Application number: 82400452.7

(22) Date of filing: 12.03.82

(51) Int. Cl.³: C 07 C 31/20, C 07 C 29/136

(30) Priority: 12.03.81 US 239764

(43) Date of publication of application: 22.09.82 Bulletin 82/38

(84) Designated Contracting States: AT BE DE FR GB IT NL SE

(71) Applicant: UNION CARBIDE CORPORATION, Old Ridgebury Road, Danbury Connecticut 06817 (US)

(72) Inventor: Poppelsdorf, Fedor, 635 Creystone Road, Charleston West Virginia 25314 (US)

(74) Representative: Rinuy, Guy et al, Cabinet Rinuy et Santarelli 14, Avenue de la Grande Armée, F-75017 Paris (FR)

(54) **Process for the preparation of ethylene glycol.**

(57) A process for the preparation of ethylene glycol by the vapor phase hydrogenation of oxalate esters whereby not more than 1.0 percent by weight of 1,2-butanediol is formed.

EP 0 060 787 A1

# PROCESS FOR THE PREPARATION OF ETHYLENE GLYCOL

This invention relates to the preparation of ethylene glycol by the vapor phase hydrogenation of oxalate esters.

The hydrogenation of alkyl esters over copper chromium oxide (copper chromite) at 200°C. to 250°C. to give the corresponding alkanols by the reaction:

$$RCOOR' + 2H_2 \xrightarrow{CuCrO_2} RCH_2OH + R'OH$$

is well known. (See: "Reactions of Hydrogen with Organic Compounds over Copper-Chromium Oxide and Nickel Catalysts," by Homer Burton Adkins, The University of Wisconsin Press (1937).)

Several prior art processes have been proposed for the preparation of alcohols and glycols by hydrogenation of certain esters of monobasic and higher dibasic acids in the liquid phase and esters of hydroxy acetic acid in vapor phase reaction systems.

Of interest is an article by H. Adkins, in R. Adams et al, ed., Organic Reactions, Vol. VIII, Chapter 1, John Wiley and Sons, Inc. New York, 1954, pp. 1-27 which sets forth a general mechanism for the liquid phase hydrogenation of esters to alcohols. It is noted therein that in the liquid phase, diethyl oxalate gives a good yield of ethylene glycol but only with a pressure much higher than normal liquid phase hydrogenation pressures, i.e., higher than 4000 psi.

U.S. Patent No. 2,060,880 discloses a process for the hydrogenation of di-alkyl esters of oxalic acid at a pressure in excess of 200 atmospheres over a copper chromite catalyst to produce ethylene glycol. Other esters of oxalic acid may also be employed.

U.S. Pat. No. 2,305,104 discloses a process for the vapor phase hydrogenation of alkyl esters of hydroxy acetic acid utilizing a dual catalyst charged reaction zone at temperatures between 150°C., and 300°C., and pressures from 10 to 1000 atmospheres or higher.

British Pat. Nos. 555,240 and 575,380 disclose processes for the vapor phase catalytic hydrogenation of hydroxy acetic acid and its derivatives (esters) and an ester of glycollic acid respectively at temperatures ranging from 150°C. to 300°C., and pressures of from 10 to 1000 atmospheres to produce ethylene glycol.

U.S. Pat. No. 4,112,245 discloses a process for the preparation of ethylene glycol by hydrogenation of oxalate esters in the vapor phase wherein the process is characterized by having a sulfur content in said oxalate ester less than 0.4 ppm and having an essentially sulfur free hydrogen in the presence of a copper-containing hydrogenation catalyst.* Although this patent states at column 4, lines 39 to 43 that

> "By the process of the present invention hydrogenolysis including the attendant problem of by-product formation on the catalyst surface, as well as poisoning and degradation of and loss of catalyst activity from the feed materials is substantially minimized",

it is clear from the several examples that by-product formation is present in significant quantities as indicated by the formation of substantial amounts of

---

*For a discussion on the effect of sulfur or chlorine containing compounds on the rate of hydrogenation of carboxylate esters, see "Reactions of Hydrogen with Organic Compounds over Copper-Chromium Oxide and Nickel Catalysts" by Homer Burton Adkins, The University of Wisconsin Press, (1937) at Page 22.

water in the examples.

The patent discloses the water formed for examples IV to X as follows:

| Example[3] | Water formed[1] |
|---|---|
| IV | 0.6 to 1.7 |
| V | 0.83 to 1.58 |
| VI | 3[2] |
| VII | 0.95 to 1.76 |
| VIII | 1.35 to 1.81 |
| IX | 0.90 to 1.65 |
| X | 0.48 to 1.02 |

[1]The water formed is given in the patent as a range based on the weight percent of water formed.

[2]No range given for example VI.

[3]Examples 1 to 3 of the patent are comparative examples showing the effect of sulfur on yield of ethylene glycol.

The disclosure of U.S. Pat. No. 4,112,245 does not explain the relationship of the water formed in these examples and the hydrogenation process described therein (the examples V to X may not be reproduced since the catalyst used therein has not been possible to reproduce from the disclosure), nor does the patent correlate the water formed in the process to by-product formation, e.g., by-products such as 1,2-butanediol. It has been observed by applicants that by-product formation, particularly the formation of 1,2-butanediol, is substantial when the water formed in the process is about 0.5 percent by weight based on the total weight of products formed.

By reference to Table IV (examples 4 to 15) of the instant application, as set forth hereinafter, the relationship of the measured water in the hydrogenation

process to the amount of 1,2-butanediol formed is shown as follows:

| Example | Water[1] | 1,2-butanediol[1,2] | HOH/1,2-butanediol |
|---|---|---|---|
| 4 | 3.38 | 1.85 | 1.83 |
| 5 | 2.75 | 2.09 | 1.31 |
| 6 | 0.19 | nil | -- |
| 7 | 0.28 | nil | -- |
| 8 | 0.20 | nil | -- |
| 9 | 0.25 | nil | -- |
| 10 | 0.85 | 0.67 | 1.27 |
| 11 | 2.27 | 1.29 | 1.76 |
| 12 | 1.79 | 1.09 | 1.64 |
| 13 | 0.31 | nil | -- |
| 14 | 0.24 | nil | -- |
| 15 | 1.06 | 0.98 | 1.08 |

[1]weight percent based on products formed.

[2]nil indicates none detected.

The foregoing suggests that 1,2-butanediol content tracks water content and is a predominant impurity when the water content is about 1 weight percent.

Typical of other reaction by-products which can be formed during hydrogenation of dialkyl oxalates are: water; methanol; ethanol; ethylene glycol monoacetate; Cellosolve (TM); 1,2-butanediol; 2-hydroxymethyl-1,3-dioxolane; 2-methyl-1,3-dioxolane; 2-methyl-4-ethyl-1,3-dioxolane; ethyl glycolate; and diethylene glycol. The presence of asymmetrical diols, especially 1,2-butanediol is highly undesirable and their presence is to be avoided.

The ability to keep the formation of a by-product below a predetermined level of formation is of particular interest, owing to the detrimental effect that a small quantity may have on the use of the

ethylene glycol wherein it is contained. Of paramount commercial interest is the use of ethylene glycol in the manufacture of polyester fiber. When ethylene glycol contains small quantities of impurities, such as 1,2-butanediol, the overall properties of the polyester produced are changed. Thus, properties such as the dying characteristics of the polyester fiber, strength of the polyester fiber, color and the like, change in relation to the impurities present in the ethylene glycol employed in the manufacture of the polyester fiber.

Since the manufacture of polyester fiber cannot reasonably tolerate even small variances in the properties of the produced polyester fiber, the ethylene glycol used in its manufacture must have a certain constant level of purity to insure that the chemical and physical properties of the polyester fiber are relatively constant. Unfortunately, some impurities are extremely difficult to remove, yet ethylene glycol containing even minute quantities of such impurities, such as up to 1.0 percent by weight, is rendered commercially unusable. For example, if the impurity is 1,2-butanediol, the use of standard separation techniques is difficult. For example, if distillation is employed for separation, the separation is difficult owing to the fact that the boiling point of 1,2-butanediol at atmospheric pressure is about 192°C., whereas ethylene glycol has a boiling point of about 198°C. In addition, this asymmetrical diol is a highly undesirable component in ethylene glycol owing to the

effect it has the characteristics of polyester fiber made from ethylene glycol containing it. Thus, unless the formation of 1,2-butanediol is not more than about 1.0 percent by weight, viz. 0.1 to 1.0 weight percent, the resulting ethylene glycol will be, for all practical purposes, unusable for use in the manufacture of polyester fiber, owing to the necessity of employing special purification processes, and thus will result in the use of such ethylene glycol for applications requiring lesser purity. This will necessarily decrease the commercial value of such ethylene glycol.

Owing to the significant problem arising from the presence of 1,2-butanediol in the ethylene glycol formed from the hydrogenation of oxalate esters, it is desirable to have a process whereby the formation of 1,2-butanediol is not more than 1.0 percent by weight.

## SUMMARY OF THE INVENTION

The instant process is for preparing of ethylene glycol containing not more than 1.0 percent by weight 1,2-butanediol which comprises reacting a stream of a vaporous mixture of hydrogen and an alkyl oxalate having the formula:

$$RO-\overset{O}{\overset{\|}{C}}-\overset{O}{\overset{\|}{C}}-OR$$

wherein R is methyl or ethyl. The molar ratio of hydrogen to dialkyl oxalate in the reaction is greater than 4:1 and is at a pressure of between about 15 psia and about 1000 psia. The temperature is between about 180°C. and about 240°C., with a space velocity (SV)between about 3000 $hr^{-1}$ and about 25,000 $hr^{-1}$

and a liquid hourly space velocity (LHSV) of between about 0.001 $hr^{-1}$ and about 5.0 $hr^{-1}$. The process is carried out over an effective amount of a solid copper-containing hydrogenation catalyst. The process of this invention correlates the process conditions, i.e., temperature, pressure, SV and LHSV and molar ratio of hydrogen to dialkyl oxalate, such that not more than 1.0 percent by weight 1,2-butanediol is formed.

## DESCRIPTION OF THE INVENTION

The invention relates to the preparation of ethylene glycol essentially free of deleterious amounts of 1,2-butanediol by the hydrogenation of methyl or ethyl oxalate in the vapor phase at elevated temperature in the presence of a copper-containing hydrogenation catalyst.

The hydrogenation reaction is believed to proceed as follows:

$$RO-\overset{O}{\overset{\|}{C}}-\overset{O}{\overset{\|}{C}}-OR + 4H_2 \xrightarrow{\text{Catalyst}} HOCH_2CH_2OH + ROH$$

ethylene glycol     alcohol

wherein R is methyl or ethyl.

The reaction is believed to proceed stepwise according to equations (2) and (3) as follows:

(2) $(COOR)_2 + 2H_2 \longrightarrow HOCH_2CO_2R + ROH$

(3) $HOCH_2CO_2R + 2H_2 \longrightarrow HOCH_2CH_2OH + ROH$

wherein R is as previously defined.

As aforementioned, the oxalate esters which may be employed in the process of the invention have the

general formula:

$$RO-\overset{O}{\overset{\|}{C}}-\overset{O}{\overset{\|}{C}}-OR$$

wherein R is methyl or ethyl. The oxalate esters are preheated and vaporized, as may be determined by their vapor pressure, to insure that essentially all of the ester is in the vapor state when passed, along with hydrogen, over the catalyst bed. The catalyst bed is maintained at a temperature high enough to prevent condensation of the oxalate ester or the product ethylene glycol.

In carrying out the hydrogenation of the oxalate ester, hydrogen is employed in excess of the stoichiometric amount required to convert the oxalate ester to ethylene glycol and the corresponding alcohol. Thus, the molar ratio of hydrogen to alkyl oxalate is greater than 4:1. The preferred molar ratio is between about 20:1 and about 135:1 and most preferably between about 30:1 and about 70:1. Higher or lower ratios of hydrogen to oxalate ester may be employed in the process provided the alkyl oxalate is in the vapor state and hydrogen is employed in the process in at least the stoichiometric ratio of hydrogen to alkyl oxalate of 4:1. Further, as long as greater than the stoichiometric amount of hydrogen is employed, a molar amount of inert gas, i.e., inert in the hydrogenation process, such as methane or nitrogen, may be provided such that the combined molar ratio of hydrogen and inert gas (e.g. methane or nitrogen) to the oxalate ester is between about 20:1 and about 135:1 and preferably between about

30:1 and about 70:1, although other molar ratios may be employed so long as the hydrogen to oxalate ratio is greater than 4:1.

The hydrogenation catalysts employed in the process of the invention have been generally described in the prior art and any known solid copper-containing hydrogenation catalyst, or mixture of solid copper-containing catalysts, useful for the conversion of esters to alcohols may be employed.

In general, hydrogenation catalysts containing copper either in the elemental form or combined with oxygen, as well as other hydrogenating metal oxides employed in conjunction with copper, supported or unsupported, may be used. Preferred catalysts are the copper zinc chromite or copper chromite catalysts which may be promoted with barium or sodium hydroxide and which have been reduced or partially reduced with hydrogen.

Representative hydrogenation catalysts suitable for use in this invention include, for example, in addition to those above noted, zinc, copper, cadmium, chromite catalyst, copper ammonium chromate and zinc chromium oxide so long as at least some amount of a solid copper-containing catalyst is present. Other catalysts which may be suitable for use in the process comprise compositions of tin, silver, cadmium, ruthenium, zinc or lead and oxides of these metals containing chromium in admixture with solid copper-containing catalysts. Many suitable copper-containing hydrogenation catalysts are commercially available such

as copper zinc chromite catalyst, copper barium chromite catalyst, sodium hydroxide-promoted copper chromite and copper chromite catalysts such as Calsicat's (TM) Code E-103TR (Calsicat (TM) is a division of Mallinckrodt, Inc.).

The relative activity of a given solid copper-containing hydrogenation catalyst may be determined by the rate (in gram moles $liter^{-1}$ $hour^{-1}$) at which it produces the product ethylene glycol over a fixed period of time. A lower activity for a given catalyst is indicated by a lower rate relative to the rate of the catalyst as measured prior to any loss of activity from use in the process.

In carrying out the examples, set forth hereinafter, a copper chromite hydrogenation catalyst, Calsicat (TM) Code No. E-103TR, was employed. The catalyst is purchased as a pellet and then crushed such that the catalyst employed for hydrogenation has an irregular shape and a particle size between about 8 to about 14 mesh (U. S. Standard). This catalyst is partially reduced and stabilized, i.e., has at least a portion of the copper in the zero oxidation state, and has the following typical properties:

| | |
|---|---|
| Copper content as CuO (weight %) | 47.0 |
| Chromium content as $Cr_2O_3$ (weight %) | 47.0 |
| Crush strength (lbs.) | 20 |
| Surface area ($M^2$/g) | 90-100 |
| Bulk density (lbs. /ft) | 90-95 |

In the examples, the catalyst was diluted with a solid inert diluent of approximately an equal amount by volume. The solid inert diluent was an alpha-alumina which has a relatively low surface area, a shape and

size similar to that of the copper chromite catalyst and possessed the following typical properties:

| Chemical Composition | Weight Percent |
|---|---|
| Alpha-Alumina | 98.5 |
| Silicon Dioxide | 0.74 |
| Calcium Oxide | 0.22 |
| Sodium Oxide | 0.16 |
| Ferric Oxide | 0.14 |
| Potassium Oxide | 0.04 |
| Magnesium Oxide | 0.03 |
| **Physical Properties** | |
| Surface Area[1] | 0.3 $m^2/g$ |
| Pore Volume[2] | 0.50 cc/g |
| Packing Density[3] | 0.70 g/ml |
| Median Pore Diameter[4] | 21 microns |

---

[1]Method of measurement described in "Adsorption, Surface Area and Porosity", S.J. Gregg and K.S.W. Sing, Academic Press (1967), pages 316-321.

[2]Method of Measurement as described in ASTM C20-46.

[3]Calculated value based on conventional measurement of the weight of the carrier in a known volume container.

[4]Method of Measurement described in "Application of Mercury Penetration to Materials Analysis", C. Orr Jr., Powder Technology, Vol. 3, pp. 117-123 (1970).

Since the described solid copper-chromite catalyst was partially reduced by the manufacturer, the catalyst was reduced further under hydrogen as follows:

(a) the catalyst was purged with nitrogen at 10 psia to remove residual oxygen;

(b) a gaseous stream having 0.5 mole percent hydrogen in nitrogen was passed over the catalyst for a period of about 3 hours after having heated the catalyst to about 140°C. at a rate of from about 28°C. to about 56°C. per hour;

(c) the hydrogen content of the gaseous

stream was increased from 0.5 mole percent to pure hydrogen as the temperature was increased to the operating temperature at the rate set forth in (b) above. The increase in hydrogen content in the gaseous stream is at such a rate that no or a minimal exotherm is observed. If such an exotherm is observed, the hydrogen content of the gaseous stream is decreased until the exotherm subsides. The increase in hydrogen content of the gaseous stream is then resumed; and

(d) the reduced catalyst, after having been used in the process for a period of time was regenerated by passing hydrogen over the catalyst at about 218°C. or the reaction temperature for about 15 hours.

As aforestated, the instant process is advantageous in that by correlating the process conditions, the formation of 1,2-butanediol is minimized such that the concentration is less than 1.0 percent by weight, preferably less than about 0.5 percent by weight, and most preferably less than about 0.1 percent by weight, based on the weight of ethylene glycol. As will be clear from the examples and as pointed out above, the amount of 1,2-butanediol formed in the process can be related to the amount of water formed in the process.

In carrying out the process of the invention, the temperature of the hydrogenation catalyst is generally maintained between about 180°C. and about 240°C., preferably between 190°C. and about 210°C., and most preferably between about 200°C. and about 210°C.

The selection of temperature is such that it is chosen between about 180°C. and about 240°C. so that the formation of 1,2-butanediol in the process can be less than 1.0 percent by weight of products by correlating the temperature, pressure, space velocity and liquid hourly space velocity.

The pressure employed in the process is not narrowly critical and is generally between about 15 psia and about 1000 psia, preferably between about 100 psia and about 1000 psia and most preferably between about 500 psia and about 1000 psia.

In describing the process, it is desirable for the sake of brevity to refer to the space velocity as SV and liquid hourly space velocity as LHSV. The SV and LHSV are defined herein as:

SV (Space Velocity), $Hr^{-1}$ =

$$\frac{\left(F \times \frac{273}{273 + T} + \frac{W}{H} \times \frac{1}{M} \times 22.4\right)}{V} \times 1000$$

where:

F = Average gas flow rate, (liter/hour)
T = Temperature of gas feed (°C.)
H = Elapsed time (hours)
W = Weight of oxalate (total grams)
M = Mole wt. of oxalate (146.14 for ethyl oxalate and 118.08 for dimethyl oxalate)
V = Volume of catalyst (cubic centimeters)

and LHSV = (Liquid hourly space velocity), $Hr^{-1}$ =

$$\frac{LV}{H} \times \frac{1}{V}$$

where:

LV = Total volume of oxalate feed (cubic centimeters).

In addition, the terms yield and conversion are used to refer to:

$$\text{Yield (Percent)} = \frac{\text{(Moles of ethylene glycol formed)}}{\text{(Moles of dialkyl oxalate fed)}} \times 100$$

Conversion (as a percent for diethyl oxalate) =

$$100 - [(A \times 1.404) + B]\, W_2/W_1$$

where for diethyl oxalate:

A = Weight, percent of ethyl glycolate in liquid product.

B = Weight, percent, of diethyl oxalate in liquid product.

$W_1$ = Weight of liquid feed (grams).

$W_2$ = Weight of liquid product (grams).

The efficiency of the process is calculated by the equation:

$$\text{Efficiency (to ethylene glycol)} = \frac{\text{Yield (Percent)}}{\text{Conversion (Percent)}} \times 100$$

The process is carried out with a space velocity of between about 3000 $hr^{-1}$ and about 25,000 $hr^{-1}$ and preferably between about 8,000 $hr^{-1}$ and about 18,000 $hr^{-1}$. The liquid hourly space velocity is generally between about 0.001 $hr^{-1}$ and about 5.0 $hr^{-1}$ and preferably between about 1.0 $hr^{-1}$ and about 3.0 $hr^{-1}$.

In carrying out the examples purified diethyl oxalate and dimethyl oxalate feedstocks were used such that the feedstock was essentially sulfur and chlorine free. The phrase "essentially sulfur and chlorine free" is meant to refer to feedstocks that contain less than about 0.4 ppm each of sulfur and chlorine and sulfur and chlorine containing compounds. A convenient method for removing such impurities from diethyl oxalate and dimethyl oxalate is set forth in U.S.P. 4,112,245 in column 5, lines 19 to 29, which disclosure is

incorporated herein by reference. The use of an essentially sulfur or chlorine free system has been discussed above in the discussion of the prior art.

Further, the hydrogen used in the process is similarly purified by a conventional gas scrubbing technique such that less than about 0.4 ppm each of sulfur and chlorine and sulfur and chlorine containing compounds are present. Typical of such conventional methods is the passing of the hydrogen through a bed of a mixture of $Fe_2O_3$ and fly ash, or through a bed of CuO/ZnO.

The process may be carried out in any suitable reactor such as a continuous-flow tubular reactor, wherein the oxalate ester is heated to the vapor state and admixed with hydrogen at the desired temperatures and pressures in the presence of a copper-containing hydrogenation catalyst which may be in the form of a fixed or dynamic (e.g., fluidized) catalyst bed. The vapor phase hydrogenation reaction is exothermic in nature and the tubular reactor, if employed, is preferably cooled by external cooling means to maintain the reaction temperature in the desired range. The vaporous reaction products may be recovered and treated by any conventional process, e.g., distillation, and since the process is preferably carried out in a continuous manner said recovery process will preferably include recycling of hydrogen and like reusable components, such as the oxalate reactant.

In the examples which follow the hydrogenation runs were carried out in a 1 inch (inside diameter)

straight through tube reactor 3 feet 10 inches in length, equipped with an external heating jacket (3300 watt winding) to bring the catalyst bed to reaction temperature. A hydrogenation catalyst bed (with solid inert dilutent) as described above was positioned in the middle of the reactor tube and held in position with porous glass wool plugs. An electrically heated tube packed with Filtros (TM) packing was used to vaporize the oxalate ester prior to introduction into the hydrogenation reactor. Nitrogen and hydrogen were preheated to the reaction temperature in an electrically-heated tube packed with Filtros (TM) packing. The vaporous product and by-product effluent from the reactor were conducted into a straight through standard water cooled (tempered water) coil-type condenser installed down stream from the reactor and then into a liquid/gas separator.

The condensate collected in the coil-type condenser is degassed by gravity feeding it to a high-pressure separator followed by a low-pressure separator. Liquid and gaseous samples are then taken for analysis.

The liquid reaction products were analyzed by gas chromatography using a 10 foot x 1/8 inch stainless steel column packed with TENAX (TM) GC 60/80 mesh, using helium as the carrier gas (15 ml/min) with an injector temperature of about 90°C. to about 320°C. with the temperature increasing at about 8°C. per minute. A thermal conductivity detector maintained at 300°C. was used for detection. The following are typical retention

times observed for the following compounds:

| Compound | Retention on Time (min.) |
|---|---|
| Water | 1.63 |
| Methanol | 2.58 |
| Ethanol | 3.67 |
| n-Propanol | 6.68 |
| Ethylene glycol | 10.09 |
| Dimethyl oxalate | 12.25 |
| 1,2-Butanediol | 12.87 |
| Ethyl glycolate | 14.19 |
| Diethyl oxalate | 16.69 |

The gaseous reaction products were analyzed on a continuous basis by a Vapor Fraction Analyzer (VFA) supplied by solenoid driven demi-valves and three separate analyzers and detectors. Two analyzers were equipped with thermal conductivity detectors and the third analyzer was equipped with a photometric flame-ionization detector. In each case, the quantitation was by peak height measurement. The carrier gas was hydrogen (50 psia) for the analyzer used for hydrogen detection.

The following examples are provided to illustrate the present invention in accordance with the above, but are not to be construed as limiting the invention in any way.

EXAMPLES 1-3

Examples 1-3 were carried out by charging 103 cubic centimeters of a copper chromite catalyst comprising 53 cubic centimeters of Calsicat (TM) copper chromite (Code E-103TR), prepared as above described, and 50 cubic centimeters of the solid inert diluent above described, in a tube reactor, as above described.

Dimethyl oxalate and hydrogen, containing less than about 0.4 ppm sulfur and chlorine, were preheated at about 200°C., thereby vaporizing the dimethyl oxalate. The vaporized oxalate/hydrogen mixture was introduced into the reactor and over the solid copper-containing catalyst. Table I sets forth reaction conditions and some results for Examples 1 to 3.

TABLE I

| | Example 1 | Example 2 | Example 3 |
|---|---|---|---|
| Reaction temp. (°C.) | 218 | 218 | 217 |
| Pressure ($H_2$, psia) | 450 | 450 | 450 |
| SV ($hr.^{-1}$) | 18,824 | 19,278 | 19,423 |
| LHSV ($hr.^{-1}$) | 0.83 | 0.83 | 0.90 |
| Duration of run (hr.) | 16 | 16 | 12 |
| Total time catalyst in use (hr.) | 18.5 | 37.5 | 53.8 |
| Yield[1] (ethylene glycol) | 87.9 | 85.6 | 74.1 |
| Conversion (dimethyl oxalate) | 98.7 | 92.3 | 86.0 |
| Efficiency (ethylene glycol) | 89.0 | 92.8 | 86.2 |
| Yield[1] (methyl glycolate) | 1.22 | 7.1 | 11.5 |
| Yield[1] (1,2-butanediol) | 0.86 | 0.31 | 0.33 |
| Water[2] | 1.95 | 1.31 | 1.26 |
| Rate (moles $l^{-1}$ $hr.^{-1}$) | 6.75 | 6.48 | 6.22 |

[1]All yields are expressed as a percentage, as previously set forth.

[2]Weight percent based on total weight of products.

The liquid and gaseous reaction products of examples 1-3 were analyzed according to the previously described procedure, giving the results set forth in Table II.

TABLE II[1]

| Compound | Example 1 | Example 2 | Example 3 |
|---|---|---|---|
| Methanol | 34.65 | 34.89 | 30.56 |
| Ethanol | 1.39 | 0.61 | 0.43 |
| Unknown | 0.19 | 0.32 | 0.31 |
| Water | 1.95 | 1.31 | 1.26 |
| Unknown | 0.03 | 0.02[4] | 0.01[4] |
| Methyl CELLOSOLVE™ | 0.36 | 0.33 | 0.32 |
| Methyl glycolate | 1.22 | 6.58 | 11.39 |
| Dimethyl oxalate | Trace | 0.72 | 3.25 |
| Propylene glycol[3] | 0.05[4] | 0.05[5] | 0.05[4] |
| Ethylene glycol | 58.69 | 54.40 | 51.61 |
| 1,2-butanediol | 0.86 | 0.29 | 0.33 |
| Diethylene glycol | 0.63 | 0.52 | 0.46 |
| Unknown | nil[5] | nil[5] | 0.06 |
| 2-hydroxymethyl-1, 3-dioxolane[3] | 0.87 | 0.80 | 0.82 |

[1]Most results were obtained on a CARBOWAX 20M (TM) column.

[2]Estimated from OV-101 column using REGISIL (TM) derivative method.

[3]Result from Tenax (TM) column method. Note: this component will elute with ethylene glycol on a CARBOWAX 20M (TM) column.

[4]Approximate values owing to low concentration.

[5]nil indicates none of this product was detected.

EXAMPLES 4-15

These examples were carried out as set forth in examples 1 to 3 except that diethyl oxalate was employed and the reaction time was as shown in Table III, the SV ($hr.^{-1}$) as shown in Table III, the LHSV ($hr.^{-1}$) was

about 1.05 $hr.^{-1}$, the pressure of hydrogen was varied as shown in Table III and the molar ratio of hydrogen to diethyl oxalate was as shown in Table III.

The formation of impurities were observed to be minimized with no 1,2-butanediol being detected when less than about 0.31 percent by weight water was detected. The results of these examples are set forth in Table III. Table IV sets forth the results of the analysis of the liquid and vapor streams for examples 4-15.

TABLE III[1]

| Example | Pressure ($H_2$) Psig | Temp. °C | Mole Ratio $H_2$/Diethyl Oxalate | Space Velocity ($Hr^{-1}$) | Efficiency to Glycol[6] | Conversion[7] | Rate[8] | 1,2-Butanediol[5], Yield, % |
|---|---|---|---|---|---|---|---|---|
| 4[4] | 1000 | 240 | 128 | 22,704 | 70.4 | 98.8 | 5.40 | 2.95 |
| 5[4] | 200 | 240 | 62.4 | 11,457 | 73.6 | 98.1 | 5.80 | 3.22 |
| 6 | 1000 | 200 | 72.6 | 12,422 | 88.0 | 76.5 | 5.08 | nil |
| 7[2] | 1000 | 200 | 63.0 | 10,665 | 83.2 | 93.8 | 5.87 | nil |
| 8 | 200 | 200 | 130 | 23,383 | 86.3 | 32.8 | 2.25 | nil |
| 9[2] | 200 | 200 | 124 | 22,506 | 95.5 | 77.6 | 5.95 | nil |
| 10[3] | 450 | 218 | 102 | 18,250 | 88.3 | 99.4 | 6.96 | 1.08 |
| 11[4] | 1000 | 240 | 64.0 | 11,364 | 73.2 | 99.2 | 5.66 | 2.09 |
| 12[4] | 200 | 238 | 131 | 23,701 | 88.1 | 97.6 | 6.88 | 1.58 |
| 13 | 1000 | 200 | 135 | 23,413 | 96.5 | 97.8 | 7.23 | nil |
| 14 | 200 | 200 | 64.0 | 11,297 | 90.4 | 64.5 | 4.55 | nil |
| 15[3] | 450 | 218 | 100 | 18,260 | 89.2 | 99.1 | 6.72 | 1.55 |

[1] Examples 4-15 use diethyl oxalate as the dialkyl oxalate.
[2] Example is the same as the immediately preceding example as to process conditions.
[3] Example carried out as a standard to determine if catalyst regeneration required.
[4] Comparative example.
[5] nil indicates that no 1,2-butanediol was detected.
[6] Efficiency to ethylene glycol.
[7] Conversion based on diethyl oxalate converted to product.
[8] Rate to ethylene glycol in mole $Liter^{-1}$ $hour^{-1}$.

TABLE IV[1]

Liquid Stream Analysis, (Weight Percent)[5]

| Compound | 4[4] | 5[4] | 6 | 7[2] | 8 | 9[2] | 10[3] | 11[4] | 12[4] | 13 | 14 | 15[3] |
|---|---|---|---|---|---|---|---|---|---|---|---|---|
| $H_2O$ | 3.38 | 2.75 | 0.19 | 0.28 | 0.20 | 0.25 | 0.85 | 2.57 | 1.79 | 0.31 | 0.24 | 1.06 |
| Methanol | 0.38 | 0.38 | nil | nil | nil | nil | 0.05 | 0.17 | 0.15 | 0.02 | nil | 0.07 |
| Ethanol | 61.23 | 57.14 | 53.78 | 60.75 | 20.06 | 45.03 | 58.36 | 62.79 | 52.38 | 57.71 | 43.66 | 57.65 |
| Ethylene glycol | 29.99 | 32.2 | 28.78 | 34.42 | 13.92 | 34.4 | 37.64 | 30.89 | 40.75 | 40.21 | 25.58 | 38.63 |
| Ethyl glycolate | 0.71 | 1.05 | 16.13 | 4.57 | 29.8 | 15.55 | 0.31 | 0.33 | 1.70 | 1.58 | 22.13 | 0.38 |
| 1,2-Butanediol | 1.85 | 2.09 | nil | nil | nil | nil | 0.67 | 1.29 | 1.09 | nil | nil | 0.98 |
| Diethyl oxalate | 0.24 | 0.48 | 1.00 | nil | 35.49 | 2.60 | 0.16 | 0.34 | 0.24 | nil | 5.61 | 0.40 |

Vapor Stream Analysis (mole percent)[6]

| | | | | | | | | | | | | |
|---|---|---|---|---|---|---|---|---|---|---|---|---|
| Ethanol | 0.133 | 0.524 | 0.138 | 0.136 | 0.374 | 0.455 | 0.21 | 0.15 | 0.698 | 0.241 | 0.571 | 0.277 |
| Methane | nil | 0.04 | 0.04 | nil | 0.04 | nil | 0.04 | 0.04 | 0.04 | 0.04 | nil | 0.04 |

[1] Examples 4-15 use diethyl oxalate as the dialkyl oxalate.

[2] Example is the same as the preceding example as to process conditions.

[3] Example carried out as a standard to determine if catalyst regeneration required.

[4] Comparative example.

[5] nil indicates this compound was not detected.

[6] Balance is substantially hydrogen.

EXAMPLE 16

This example was carried out according to the procedure of examples 1-3 under standard conditions except that the LHSV was 1.60 and diethyl oxalate was employed. This example gave a rate to ethylene glycol of 9.26 mole liter$^{-1}$ hour$^{-1}$ (35.9 lbs/ft$^3$ catalyst/hr.) compared to the rate of 6.5 mole liter$^{-1}$ hour$^{-1}$ under standard conditions as defined below. The efficiency to ethylene glycol was 98.4 percent and the conversion of diethyl oxalate was 80.8 percent. The analysis of the liquid reaction product is set forth in Table V.

"Standard conditions" is meant to refer to the following conditions for hydrogenation:

| | |
|---|---|
| Temperature (°C.) | 218 |
| Pressure ($H_2$, psig) | 450 |
| $H_2$/dialkyl oxalate ratio | 100 |
| SV (hr.$^{-1}$) | 18,000 |
| LHSV (hr.$^{-1}$) | 1.08 |

TABLE V

| Component | Weight, Percent |
|---|---|
| Water | 0.54 |
| Methanol | nil |
| Ethanol | 54.33 |
| Ethylene glycol | 34.51 |
| Ethyl glycolate | 12.44 |
| Diethyl oxalate | 2.16 |
| 1,2-butanediol | 0.05 |

EXAMPLE 17

This example was carried out as above described for examples 1-16 except that the temperature, pressure, SV and LHSV were selected as follows with no 1,2-butanediol being detected (diethyl oxalate as the dialkyl oxalate):

| | |
|---|---|
| Temperature (°C.) | 235 |
| Pressure (psig) | 450 |
| SV ($hr.^{-1}$) | 10,000 |
| LHSV ($hr.^{-1}$) | 1.55 |
| Hydrogen/diethyl oxalate ratio | 38.7 |

A conversion of diethyl oxalate of about 82.5 percent was observed with an efficiency to ethylene glycol of about 88 percent.

CLAIMS

1. The process for the preparation of ethylene glycol which comprises reacting in the vapor phase a mixture of a dialkyl oxalate having the formula

$$RO-\overset{O}{\overset{\|}{C}}-\overset{O}{\overset{\|}{C}}-OR$$

wherein R is methyl or ethyl, and hydrogen, with the molar ratio of hydrogen to dialkyl oxalate being at least 4 to 1, at a pressure of between about 15 psia and about 1000 psia, a temperature between about 180°C. and about 240°C., a space velocity between about 3000 hr.$^{-1}$ and about 25,000 hr.$^{-1}$ and a liquid hourly space velocity of between about 0.001 hr.$^{-1}$ and about 5.0 hr.$^{-1}$ over an effective amount of a solid copper-containing hydrogenation catalyst whereby that by correlating the temperature, pressure, space velocity and liquid hourly space velocity and molar ratio of hydrogen to dialkyl oxalate not more than 1.0 percent by weight of 1,2-butanediol is formed.

2. The process of claim 1 wherein the temperature is between about 190°C. and about 210°C. and preferably between about 200°C. and about 210°C.

3. The process of claim 1 whereby not more than 0.5 percent and preferably not more than 0.1 percent by weight of 1,2-butanediol is formed.

4. The process of claim 1 wherein the dialkyl oxalate is diethyl oxalate.

5. The process of claim 1 wherein the pressure is between about 100 psia and about 1000 psia, and preferably between about 500 psia and about 1000 psia.

6. The process of claim 1 wherein the space velocity is between about 8000 hr.$^{-1}$ and about 18,000 hr.$^{-1}$.

7. The process of claim 1 wherein the liquid hourly space velocity is between about 1.0 hr.$^{-1}$ and about 3.0 hr.$^{-1}$.

8. The process of claim 1 wherein the catalyst is selected from the group consisting of copper zinc chromite, copper chromite, copper barium chromite, and sodium hydroxide-promoted copper chromite catalysts, copper chromite being preferred.

9. The process of claim 8 wherein the catalyst is diluted with a solid inert support material, which is preferably an alpha-alumina.

10. The process of claim 1 wherein the hydrogenation catalyst is periodically regenerated with hydrogen at the reaction temperature.

11. The process of claim 1 wherein the molar ratio of hydrogen to dialkyl oxalate is between 20:1 and about 135:1 and preferably between about 30:1 and about 70:1.

12. The process of claim 11 wherein methane is provided in the process such that the combined molar ratio of hydrogen and methane to oxalate is between about 20:1 and about 135:1.

0060787

European Patent Office

# EUROPEAN SEARCH REPORT

Application number

EP 82 40 0452.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | DE - A1 - 2 936 700 (MONTEDISON)<br>* claim 1 *<br>& GB - A - 2 031 883<br>-- | 1 | C 07 C 31/20<br>C 07 C 29/136 |
| X,D | US - A - 4 112 245 (L.R. ZEHNER et al.)<br>* whole document *<br>---- | 1-2,<br>4-12 | |

**TECHNICAL FIELDS SEARCHED (Int.Cl. 3)**

C 07 C 29/136
C 07 C 31/20

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 04-06-1982 | KNAACK |

EPO Form 1503.1 06.78